# EUROPÄISCHE PATENTSCHRIFT

(11) **EP 2 338 043 B1**
(45) Veröffentlichungstag und Bekanntmachung des Hinweises auf die Patenterteilung: **16.05.2018**
(21) Anmeldenummer: 09765625.0
(22) Anmeldetag: 18.06.2009
(51) Int. Cl.: G01N 21/31, G01N 33/32, G01N 21/64

(54) **BESTIMMEN DES AUSHÄRTUNGSGRADES MITTELS EINER NACHWEISSUBSTANZ**
DETERMINATION OF DEGREE OF CURING USING A DETECTION SUBSTANCE
DÉTERMINATION DU DEGRÉ DE DURCISSEMENT AU MOYEN D'UNE SUBSTANCE DE DÉTECTION

(30) Priorität: 19.06.2008 DE 102008028857; 19.06.2008 DE 102008029281; 18.05.2009 DE 102009021677
(43) Veröffentlichungstag der Anmeldung: 29.06.2011
(73) Patentinhaber: Burth, Dirk, 85253 Erdweg (DE)
(72) Erfinder: Burth, Dirk, 85253 Erdweg (DE)
(74) Vertreter: SR Huebner - Munich Patentanwaltspartnerschaft mbB
(86) Internationale Anmeldenummer: PCT/EP2009/004407
(87) Internationale Veröffentlichungsnummer: WO 2009/153045

(56) Entgegenhaltungen:
- US-A- 4 651 011
- US-A- 5 556 663
- US-A1- 2003 074 095

## Beschreibung

Die Erfindung betrifft ein Verfahren zur Bestimmung eines Aushärtungsgrades einer Beschichtung auf einem Substrat.

Der Aushärtegrad spielt bei vielen Beschichtungen, beispielsweise bei reaktiven Lacken, Druckfarben, Klebstoffschichten und Silikonisierungen eine wichtige Rolle für die Eigenschaften. Es werden dadurch z.B. beeinflusst die Härte, die Abriebbeständigkeit, das Migrationsverhalten. Es ist deswegen von Bedeutung, den Aushärtungsgrad einer Beschichtung zu bestimmen. Besonders ist dies bei strahlungshärtenden Lacken und Druckfarben von Bedeutung. Hier spielt der Aushärtungsgrad insbesondere hinsichtlich des Migrationsverhaltens eine Rolle. Die bisherigen Methoden zur Bestimmung des Aushärtungsgrades geben nicht immer eindeutig Ergebnisse.

In der US-Patentanmeldung US 2003/0074095 A1 ist ein Verfahren zum Messen des Vernetzungsgrades einer Beschichtung beschrieben. Das Verfahren umfasst das Durchführen eines Beschichtungsvorgangs bei einem metallhaltigen Substrat zur Bereitstellung eines beschichteten metallhaltigen Substrats, das Positionieren einer Prüfvorrichtung nahe dem beschichteten metallhaltigen Substrat und das in Betrieb nehmen der Prüfvorrichtung, um einen Wert des Vernetzungsgrades zu bestimmen, wobei sich der Wert auf ein Gebiet des beschichteten metallhaltigen Substrats bezieht. Als Beispiel 1 der US 2003/0074095 A1 ist eine Ausführungsform beschrieben, bei der Getränkedosen mit einer Polymerbeschichtung versehen werden. Zur Bestimmung des Vernetzungsgrades der Polymerbeschichtung wird der Farbstoff Bromphenolblau zugegeben, der mit der nicht abreagierten Vernetzerfunktionalität reagiert. Abhängig von der Menge an noch nicht abreagierter Vernetzerfunktionalität ändert sich die Farbe des Bromphenolblau, welche durch eine Farbanalyse bestimmt wird.

Es ist Aufgabe der Erfindung, eine zuverlässige Bestimmung des Aushärtungsgrades einer Beschichtung auf einem Substrat zu ermöglichen.

Die Aufgabe der Erfindung wird durch ein Verfahren zur Bestimmung eines Aushärtungsgrades einer Beschichtung auf einem Substrat gemäß Anspruch 1, sowie durch ein weiteres Verfahren zur Bestimmung eines Aushärtungsgrades einer Beschichtung auf einem Substrat gemäß Anspruch 6 gelöst.

Das erfindungsgemäße Verfahren dient zur Bestimmung eines Aushärtungsgrades einer Beschichtung auf einem Substrat. Das Verfahren umfasst das in Kontakt bringen der Beschichtung während einer ersten Zeitspanne mit einem ersten Material, das eine vorgegebene Konzentration an Nachweissubstanz enthält. Darüber hinaus umfasst das Verfahren das quantitative Bestimmen einer charakteristischen Größe, die von einer während der ersten Zeitspanne in die Beschichtung eingedrungenen Menge oder Konzentration der Nachweissubstanz abhängt, mittels mindestens einer von einer optischen Untersuchung, einer spektroskopischen Untersuchung, einer chromatographischen Untersuchung der Beschichtung, sowie das Bestimmen einer Eintrittskinetik der Nachweissubstanz in die Beschichtung anhand der charakteristischen Größe, wobei eine gut gehärtete Beschichtung relativ stark vernetzt ist, so dass die Nachweissubstanz nur vergleichsweise langsam eindringen kann, wohingegen die Nachweissubstanz in eine weniger gut gehärtete Beschichtung relativ schnell eindringen kann, wobei die Eintrittskinetik einen Rückschluss auf den Aushärtungsgrad der Beschichtung erlaubt. Darüber hinaus umfasst das Verfahren das Ableiten des Aushärtungsgrades der Beschichtung aus der Eintrittskinetik der Nachweissubstanz in die Beschichtung, wobei der Aushärtungsgrad der Beschichtung umso höher ist, je geringer die Menge oder Konzentration der Nachweissubstanz ist, die während der ersten Zeitspanne in die Beschichtung eindringt.

Das erfindungsgemäße Verfahren beruht darauf, dass das Polymernetzwerk in einer ausgehärteten Beschichtung dichter ist als in einer weniger ausgehärteten Beschichtung. In einer ausgehärteten Beschichtung tritt deswegen eine Nachweissubstanz aus einer Lösung langsamer ein als in eine schlechter gehärtete Probe. Dies liegt unter anderem daran, dass das Lösungsmittel die Beschichtung weniger aufquillt, wenn diese besser ausgehärtet ist. Die Menge an in eine Beschichtung eingetretener Nachweissubstanz in einer vorgegebenen Zeitspanne ist daher ein Maß für den Aushärtungsgrad.

Gemäß einem Beispiel handelt es sich bei dem ersten Material um eine erste Lösung, in der die Nachweissubstanz enthalten ist.

Beispielsweise umfasst das Verfahren: Eintauchen der Beschichtung des Substrats in die erste Lösung, und Herausnehmen des Substrats aus der ersten Lösung nach Ablauf der ersten Zeitspanne. Durch das Herausnehmen des Substrats kann dieses durch z.B. Abputzen gereinigt werden. Dies hilft, damit nur die Nachweissubstanz in der Beschichtung bestimmt werden und nicht eventuell Nachweissubstanz aus der Lösung, die sich noch auf der Beschichtung befindet. Das Bestimmen der Nachweissubstanz in der Beschichtung ist generell einfacher, wenn die Beschichtung sich nicht mehr in der Lösung befindet.

Gemäß einem Ausführungsbeispiel umfasst die erste Lösung mindestens eines der folgenden Lösungsmittel: Wasser, Ethanol, Methanol, Isopropanol, Aceton, Essigester, Chloroform. Es können auch Gemische dieser Lösungsmittel eingesetzt werden. Diese Lösungsmittel lösen zum einen die Nachweissubstanz gut. Weiterhin können diese Lösungsmittel Beschichtungen unterschiedlich stark anquellen. Je stärker die Anquellung, desto schneller tritt das Nachweisreagens in die Beschichtung ein. Durch die Wahl des Lösungsmittels kann damit eingestellt werden, wie lange man warten muss, bis die Nachweissubstanz in der Beschichtung ist. Damit kann insgesamt die Geschwindigkeit des Nachweisverfahrens beeinflusst werden.

Zum Beispiel handelt es sich bei dem ersten Material um mindestens eines der folgenden: ein viskoelastisches Material, ein hochviskoses Material, eine Knetmasse, einen Haftklebstoff, eine Haftklebemasse, einen Haftklebestreifen mit einem Haftklebstoff, ein Haftklebeband mit einem Haftklebstoff, ein Haftetikett mit einem Haftklebstoff.

Beispielsweise umfasst das erste Material mindestens eines der folgenden: Acrylat, Polyvinylpyrolidon, Polyäthylenglykol, Harz sowie Abmischungen davon.

Gemäß einer vorteilhaften Ausführungsform der Erfindung ist die charakteristische Größe ein Maß für eine Menge oder Konzentration der Nachweissubstanz in der Beschichtung nach Ablauf der ersten Zeitspanne. Da die Menge oder Konzentration der Nachweissubstanz Rückschlüsse auf den Aushärtungsgrad erlaubt, ist es von Vorteil, diese Größe zu bestimmen.

Gemäß einer vorteilhaften Ausführungsform der Erfindung wird die charakteristische Größe mehrfach für verschiedene Werte der ersten Zeitspanne bestimmt. Die mehrfache Bestimmung ergibt genauere Messwerte. Außerdem erniedrigt eine Mehrfachmessung die Gefahr, durch eine zufällige Fehlmessung den Aushärtungsgrad ungenau zu bestimmen. Weiterhin können bei Mehrfachmessungen die einzelnen Punkte zu einer Kurve verbunden werden. Messkurven erlauben eine eindeutigere Zuordnung der Messdaten zu einem Härtungsgrad. Die dadurch erhaltene Sicherheit ist zum Beispiel besonders im Verpackungsbereich von Bedeutung, bei dem eine ausreichende Aushärtung extrem wichtig ist, damit kein Übergang von Beschichtungsbestandteilen auf ein z.B. Lebensmittel erfolgt.

Gemäß einer vorteilhaften Ausführungsform der Erfindung wird die charakteristische Größe als Funktion der ersten Zeitspanne bestimmt. Funktionen erlauben eine eindeutigere Zuordnung der Messdaten zu einem Härtungsgrad. Die dadurch erhaltene Sicherheit ist zum Beispiel besonders im Verpackungsbereich von Bedeutung, bei dem eine ausreichende Aushärtung extrem wichtig ist, damit kein Übergang von Beschichtungsbestandteilen auf ein z.B. Lebensmittel erfolgt.

Erfindungsgemäß wird anhand der charakteristischen Größe eine Eintrittskinetik der Nachweissubstanz in die Beschichtung bestimmt. Die Eintrittskinetik der Nachweissubstanz zu bestimmen ist von Bedeutung, da diese mit dem Polymerisationsgrad oder der Netzwerkdichte in einer Beschichtung korreliert. Bei hohen Polymerisationsgraden oder Netzwerkdichten können Nachweissubstanzen nur langsam in die Beschichtung eintreten. Deswegen gibt die Eintrittskinetik eine Information über den Aushärtungsgrad. Die Eintrittskinetik lässt sich anhand der charakteristischen Größe, die ein Maß für die Menge der Nachweissubstanz in der Beschichtung ist, bestimmen. Dies kann zum Beispiel durch eine einmalige Messung nach einer bestimmten Zeit erfolgen oder durch mehrere Messungen nach verschiedenen Zeiten. Letzteres ist als Mehrfachmessung genauer als eine Einzelbestimmung.

Gemäß der Erfindung ist der Aushärtungsgrad der Beschichtung umso höher, je geringer die Menge oder Konzentration der Nachweissubstanz ist, die während der ersten Zeitspanne in die Beschichtung eindringt. Dies liegt daran, dass z.B. das polymere Netzwerk in einer ausgehärteten Beschichtung dichter ist als in einer weniger ausgehärteten Beschichtung. In einer ausgehärteten Beschichtung tritt deswegen eine Nachweissubstanz aus einer Lösung langsamer ein als in eine schlechter gehärtete Probe. Dies liegt unter anderem daran, dass ein Lösungsmittel die Beschichtung weniger aufquillt, wenn diese besser ausgehärtet ist.

Beispielsweise handelt es sich bei der Beschichtung um eine der folgenden: eine Lackschicht, eine Druckfarbenschicht, eine Klebstoffschicht, eine Silikonisierung. Bei all diesen Materialien ist der Aushärtungsgrad von Bedeutung und kann mit dem erfindungsgemäßen Verfahren bestimmt werden. Zum Beispiel ist das besonders im Verpackungsdruck von Bedeutung, da hier keine migrierfähigen Substanzen vorliegen dürfen. Migrierfähige Substanzen können nur reduziert sein, wenn z.B. der Aushärtungsgrad ausreichend ist. Eine Bestimmungsmethode auf den Aushärtungsgrad hilft zu erkennen, dass ein Herstellungsprozess richtig abläuft. Auch kann es zur Qualitätskontrolle genommen werden, um zu bestätigen dass eine Lackschicht, Druckfarbenschicht, Klebstoffschicht oder Silikonisierung den Qualitätsanforderungen genügt.

Gemäß einem Beispiel handelt es sich bei der Beschichtung um eine Schicht einer strahlungshärtenden Druckfarbe oder eines strahlungshärtenden Lackes. Strahlungshärtende Druckfarben oder Lacke sind besonders gefährdet, bei schlechter Aushärtung migrierfähige Substanzen freizusetzen. Deswegen muss der Aushärtungsgrad geprüft werden.

Beispielsweise ist die Beschichtung unter anderem in Form einer Druckmarke auf dem Substrat aufgebracht.

Gemäß einem Beispiel handelt es sich bei der Nachweissubstanz um eine der folgenden: einen Photoinitiator, einen fluoreszenzaktiven Stoff, einen farbigen Stoff, einen Farbstoff, Farbpigmente, eine UV-aktive Substanz. Fluoreszenzaktive Substanzen lassen sich in geringsten Mengen einfach nachweisen. Hierbei stören oft auch Farbpigmente die Bestimmung nicht. Die Messgeräte für die Fluoreszenzmessung gibt es auch in günstigen Ausführungsformen. Farbige Stoffe, Farbstoffe, Farbpigmente eignen sich ebenfalls besonders gut, da mit diesen bereits optisch beurteilt werden kann, wie viel Nachweissubstanz in die Beschichtung eingedrungen ist. Dadurch sind keine teuren Messgeräte nötig. Weiterhin ist die optische Beurteilung sehr schnell und einfach, was vor allem in einem Produktionsprozess hilfreich ist.

Zum Beispiel handelt es sich bei der Nachweissubstanz um Iod. Die Verwendung von Iod als Nachweissubstanz ist von Vorteil, da mit Iod bereits optisch beurteilt werden kann, wie viel Nachweissubstanz in die Beschichtung eingedrungen ist. Dadurch sind keine teuren Messgeräte nötig. Weiterhin ist die optische Beurteilung sehr schnell und einfach, was vor allem in einem Produktionsprozess hilfreich ist. Iod hat den weiteren Vorteil, dass es sehr schnell eindringen kann. Es löst sich dabei in Wasser und ist damit lösungsmittelfrei zu handhaben, was ökologisch und gesundheitlich von Vorteil ist. Iod lässt sich zudem schnell wieder aus der Beschichtung herauslösen und als Iod Stärkekomplex in geringsten Konzentrationen nachweisen.

Gemäß einem Ausführungsbeispiel wird nach Ablauf der ersten Zeitspanne zur Bestimmung der charakteristischen Größe eine Untersuchung der Beschichtung durchgeführt.

Gemäß einem Beispiel wird nach Ablauf der ersten Zeitspanne die in der Beschichtung enthaltene Menge oder Konzentration der Nachweissubstanz quantisiert. Die quantitative Bestimmung hilft, da damit auch der Aushärtungsgrad nicht nur qualitativ oder subjektiv angegeben werden kann, sondern mit einer z.B. Zahl eindeutig festgelegt ist. Dies ist z.B. wichtig, wenn klare Qualitätswerte notwendig sind z.B. wie im Verpackungsdruck anhand derer entschieden wird, inwieweit das Produkt genug ausgehärtet ist und in den Verkehr gebracht werden darf.

Gemäß der Erfindung wird zur Bestimmung der charakteristischen Größe mindestens eine von einer optischen Untersuchung, einer spektroskopischen Untersuchung, einer chromatographischen Untersuchung der Beschichtung durchgeführt. Diese Methoden lassen sich schnell anwenden und ergeben quantitative Werte, mit denen der Aushärtungsgrad beschrieben werden kann. Die Messgeräte wie z.B. optische Messgeräte wie z.B. ein Densitometer haben z.B. Drucker bereits im Einsatz und müssen nicht extra zugekauft werden.

Beispielsweise handelt es sich bei der spektroskopischen Methode um eine der folgenden: UV-Spektroskopie, Spektroskopie im sichtbaren Bereich, IR-Spektroskopie, Raman-Spektroskopie, Fluoreszenzspektroskopie, Spektralphotometrie, Weißemessung, Densitometrie. Es ist besonders vorteilhaft, wenn die UV Spektroskopie, Spektroskopie im sichtbaren Bereich, IR-Spektroskopie, Raman-Spektroskopie, Fluoreszenzspektroskopie, Spektralphotometrie, Weißemessung zur Bestimmung der aus der Beschichtung ausgetretenen Nachweissubstanz herangezogen wird. Diese Methoden erlauben sehr schnelle Messungen und quantifizieren sehr genau. Außerdem lassen sich durch diese Methoden sehr viele Substanzen vermessen. Die Methoden lassen sich besonders leicht anwenden in Beschichtungen. Bei der UV Spektroskopie, Spektroskopie im sichtbaren Bereich, IR-Spektroskopie, Raman-Spektroskopie, Fluoreszenzspektroskopie, Spektralphotometrie, Weißemessung können problemlos Messungen über einen längeren Zeitraum aufgenommen werden.

Gemäß einem Beispiel handelt es sich bei der charakteristischen Größe um eine der folgenden: eine optische Eigenschaft der Beschichtung, eine spektroskopische Eigenschaft der Beschichtung, eine chromatographische Eigenschaft der Beschichtung. Diese Eigenschaften lassen sich besonders schnell und leicht messen, da hierfür verschiedene Messgeräte zur Verfügung stehen, die auch eine Quantifizierung ermöglichen. Diese Eigenschaften eignen sich damit z.B., um eine Schnellmessmethode für den Aushärtungsgrad zu bekommen.

Beispielsweise handelt es sich bei der Nachweissubstanz um Iod, und nach Ablauf der ersten Zeitspanne wird eine Menge oder eine Konzentration von Iod in der Beschichtung bestimmt.

Gemäß einem weiteren Beispiel wird die Nachweissubstanz, die während der ersten Zeitspanne in die Beschichtung eindringt, nach Ablauf der ersten Zeitspanne wieder ganz oder teilweise aus der Beschichtung herausgelöst. Das ist von Vorteil z.B., wenn es sich bei der Beschichtung um dunkle Druckfarbe handelt, die eine spektroskopische oder optische Untersuchung der Nachweissubstanz in der Druckfarbe unmöglich macht. Es ist dann vorteilhaft, wenn die Nachweissubstanz z.B. in eine Lösung herausgelöst wird und dort die quantitative Bestimmung erfolgt. Weiterhin ist es von Vorteil, wenn die Austrittskinetik einer Nachweissubstanz leichter gemessen werden kann als deren Eindringkinetik. Durch die Kombination von Eintritt und nachfolgend Austritt wird eine Differenzierung zwischen verschiedenen Härtungsgraden größer. Bei einem schnelleren Eintritt der Nachweissubstanz befindet sich nach einer bestimmten Zeit mehr Nachweissubstanz in der Beschichtung. Dies hat zur Folge, dass der Austritt der Nachweissubstanz schneller wird erstens aufgrund seiner schnelleren Beweglichkeit in der Beschichtung und zudem wegen der größeren Menge an Nachweissubstanz, was einen höheren Konzentrationsgradient mit sich bringt.

Beispielsweise wird die charakteristische Größe nach dem Herauslösen der Nachweissubstanz aus der Beschichtung bestimmt. Die Bestimmung der charakteristischen Größe nach dem Herauslösen ist vorteilhaft, da z.B. das spektroskopische Nachweisen einer Nachweissubstanz in der Beschichtung schwierig sein könnte, z.B. wenn farbige Beschichtungen vorliegen. Daher ist es leichter, die Nachweissubstanz erst nachzuweisen, wenn diese wieder herausgelöst ist aus der Beschichtung.

Gemäß einer vorteilhaften Ausführungsform der Erfindung umfasst das Verfahren: in Kontakt bringen, nach Ablauf der ersten Zeitspanne, der Beschichtung mit einem zweiten Material während einer zweiten Zeitspanne, wobei das zweite Material dazu ausgelegt ist, Nachweissubstanz aus der Beschichtung aufzunehmen, und Bestimmen der charakteristischen Größe als Maß für eine Menge oder Konzentration der Nachweissubstanz in dem zweiten Material. Dies ist z.B. vorteilhaft, wenn das spektroskopische Nachweisen einer Nachweissubstanz in der Beschichtung schwierig sein könnte. Wenn z.B. farbige Beschichtungen vorliegen, ist es leichter, die Nachweissubstanz erst nachzuweisen, wenn diese wieder herausgelöst ist aus der Beschichtung. Die Quantifizierung unterliegt dabei weniger Fehlermöglichkeiten wie z.B. durch Absorption durch die Beschichtung. Die quantitative Bestimmung ist von Bedeutung, da damit auch der Aushärtungsgrad nicht nur qualitativ oder subjektiv angegeben werden kann, sondern mit einer z.B. Zahl eindeutig festgelegt ist. Dies ist z.B. wichtig, wenn klare Qualitätswerte notwendig sind z.B. wie im Verpackungsdruck, anhand derer entschieden wird, inwieweit das Produkt genug ausgehärtet ist und in den Verkehr gebracht werden darf.

Gemäß einem Beispiel handelt es sich bei dem zweiten Material um eine zweite Lösung, die dazu ausgelegt ist, Nachweissubstanz aus der Beschichtung aufzunehmen.

Beispielsweise ist in der zweiten Lösung anfangs im Wesentlichen keine Nachweissubstanz enthalten.

Entsprechend einem Ausführungsbeispiel umfasst das Verfahren: Lösen zumindest von Teilen der während der ersten Zeitspanne in die Beschichtung eingedrungenen Nachweissubstanz in der zweiten Lösung, und, nach Ablauf der zweiten Zeitspanne, Bestimmen der charakteristischen Größe als Maß für eine Menge oder Konzentration der Nachweissubstanz in der zweiten Lösung.

Beispielsweise ist in dem zweiten Material anfangs im Wesentlichen keine Nachweissubstanz enthalten.

Gemäß einem Beispiel handelt es sich bei dem zweiten Material um mindestens eines der folgenden: ein viskoelastisches Material, ein hochviskoses Material, eine Knetmasse, einen Haftklebstoff, eine Haftklebemasse, einen Haftklebestreifen mit einem Haftklebstoff, ein Haftklebeband mit einem Haftklebstoff, ein Haftetikett mit einem Haftklebstoff.

Beispielsweise umfasst das zweite Material mindestens eines der folgenden: Acrylat, Polyvinylpyrolidon, Polyäthylenglykol, Harz sowie Abmischungen davon.

Gemäß einem weiteren Beispiel wird nach Ablauf der zweiten Zeitspanne die in dem zweiten Material enthaltene Menge oder Konzentration der Nachweissubstanz quantisiert. Dies ist z.B. vorteilhaft, wenn das spektroskopische Nachweisen einer Nachweissubstanz in der Beschichtung schwierig sein könnte. Wenn z.B. farbige Beschichtungen vorliegen, ist es leichter, die Nachweissubstanz erst nachzuweisen, wenn diese wieder herausgelöst ist aus der Beschichtung. Eine quantitative Bestimmung einer Nachweissubstanz in Lösung ist einfacher als in einer Beschichtung.

Entsprechend einem Ausführungsbeispiel wird zur Bestimmung der charakteristischen Größe mindestens eine von einer optischen Untersuchung, einer spektroskopischen Untersuchung, einer chromatographischen Untersuchung des zweiten Materials durchgeführt. Diese Eigenschaften lassen sich besonders schnell und leicht messen, da hierfür verschiedene Messgeräte zur Verfügung stehen, die auch eine Quantifizierung ermöglichen. Diese Eigenschaften eignen sich damit z.B., um eine Schnellmessmethode auf den Aushärtungsgrad zu bekommen.

Gemäß einem Beispiel handelt es sich bei der spektroskopischen Untersuchung um eine der folgenden: UV-Spektroskopie, Spektroskopie im sichtbaren Bereich, IR-Spektroskopie, Raman-Spektroskopie, Fluoreszenzspektroskopie, Spektralphotometrie, Weißemessung, Densitometrie. Es ist besonders vorteilhaft, wenn die UV Spektroskopie, Spektroskopie im sichtbaren Bereich, IR-Spektroskopie, Raman-Spektroskopie, Fluoreszenzspektroskopie, Spektralphotometrie, Weißemessung zur Bestimmung der ausgetretene Nachweissubstanz aus der Beschichtung herangezogen wird. Diese Methoden erlauben sehr schnelle Messungen und quantifizieren sehr genau. Außerdem lassen sich durch diese Methoden sehr viele Substanzen vermessen. Die Methoden lassen sich besonders leicht anwenden in Beschichtungen. Bei der UV-Spektroskopie, Spektroskopie im sichtbaren Bereich, IR-Spektroskopie, Raman-Spektroskopie, Fluoreszenzspektroskopie, Spektralphotometrie, Weißemessung können problemlos auch Messungen über einen längeren Zeitraum aufgenommen werden, was die Genauigkeit der Messdaten erhöht.

Zum Beispiel handelt es sich bei der Nachweissubstanz um Iod, und nach Ablauf der zweiten Zeitspanne wird eine Menge oder eine Konzentration von Iod in dem zweiten Material bestimmt. Iod ist eine günstige Substanz, die einfach zu handhaben ist. Sie löst sich in verschiedenen Lösungsmitteln. Es löst sich auch gut in Wasser, zumindest nach Zugabe von Kaliumiodid. Es dringt sehr schnell in Beschichtungen ein, wobei das Eindringverhalten von der Aushärtung der Beschichtung abhängt. Unterschiede zwischen unterschiedlich gehärteten Beschichtungen lassen sich z.B. nach Zeitspannen zwischen 0,5 - 5 min erkennen. Iod ist gefärbt und lässt sich damit optisch erkennen. Das Iod hat den weiteren Vorteil, dass es sehr schnell aus der Beschichtung wieder vollständig herausgelöst werden kann. Dies ist notwendig, wenn man die Menge an Iod, die in der Beschichtung war, vollständig quantifizieren möchte. Der Nachweis des herausgelösten Iods kann in kleinsten Spuren mit Stärke erfolgen. Dies ist z.B. durch Kombination mit UV-Spektroskopie quantitativ möglich.

Gemäß einem Beispiel wird das Iod, das aus der Beschichtung herausgelöst und in dem zweiten Material enthalten ist, als Iod-Stärke-Komplex nachgewiesen.

Durch Anfärben mit Stärke bildet Iod einen blauen Komplex, der sich in geringsten Konzentrationen nachweisen lässt.

Beispielsweise wird das Verfahren zur Bestimmung eines Anteils von migrierfähigen Substanzen in der Beschichtung eingesetzt.

Ein weiteres erfindungsgemäßes Verfahren dient zur Bestimmung eines Aushärtungsgrades einer Beschichtung auf einem Substrat. Das Verfahren umfasst das in Kontakt bringen der Beschichtung während einer vorbestimmten Zeitspanne mit einem Material, wobei in der Beschichtung eine vorbestimmte Menge einer Nachweissubstanz enthalten ist, und wobei das Material dazu ausgelegt ist, Nachweissubstanz aus der Beschichtung aufzunehmen. Darüber hinaus umfasst das Verfahren das quantitative Bestimmen einer charakteristischen Größe, die von einer während der vorbestimmten Zeitspanne von der Beschichtung in das Material eingetretenen Menge oder Konzentration der Nachweissubstanz abhängt, mittels mindestens einer von einer optischen Untersuchung, einer spektroskopischen Untersuchung, einer chromatographischen Untersuchung des Materials, sowie das Bestimmen einer Austrittskinetik der Nachweissubstanz in das Material anhand der charakteristischen Größe, wobei eine gut gehärtete Beschichtung relativ stark vernetzt ist, so dass die Nachweissubstanz nur vergleichsweise langsam aus der Beschichtung austreten kann, wohingegen aus einer weniger gut gehärteten Beschichtung die Nachweissubstanz relativ schnell austreten kann, wobei die Austrittskinetik einen Rückschluss auf den Aushärtungsgrad der Beschichtung erlaubt. Darüber hinaus umfasst das Verfahren das Ableiten des Aushärtungsgrades der Beschichtung aus der Austrittskinetik der Nachweissubstanz, wobei der Aushärtungsgrad der Beschichtung umso höher ist, je geringer die Menge oder Konzentration der Nachweissubstanz ist, die während der vorbestimmten Zeitspanne in das Material eintritt.

Das erfindungsgemäße Verfahren beruht darauf, dass das Polymernetzwerk in einer ausgehärteten Beschichtung dichter ist als in einer weniger ausgehärteten Beschichtung. In einer ausgehärteten Beschichtung tritt deswegen eine Nachweissubstanz aus einer Beschichtung langsamer in eine Lösung ein als aus einer schlechter gehärteten Probe. Dies liegt unter anderem daran, dass das Lösungsmittel die Beschichtung weniger aufquillt, wenn diese besser ausgehärtet ist. Die Menge an in eine Beschichtung eingetretener Nachweissubstanz in einer vorgegebenen Zeitspanne ist daher ein Maß für den Aushärtungsgrad.

Gemäß einem Ausführungsbeispiel handelt es sich bei dem Material um eine Lösung.

Beispielsweise ist in der Lösung anfangs im Wesentlichen keine Nachweissubstanz enthalten.

Entsprechend einem Beispiel umfasst das Verfahren: Lösen zumindest von Teilen der in der Beschichtung enthaltenen Nachweissubstanz in der Lösung, und, nach Ablauf der vorbestimmten Zeitspanne, Bestimmen der charakteristischen Größe als Maß für eine Menge oder Konzentration der Nachweissubstanz in der Lösung.

Entsprechend einem weiteren Beispiel umfasst das Verfahren: Eintauchen der Beschichtung des Substrats in die Lösung, und Herausnehmen des Substrats aus der Lösung nach Ablauf der vorbestimmten Zeitspanne.

Zum Beispiel umfasst die Lösung mindestens eines der folgenden Lösungsmittel: Wasser, Ethanol, Methanol, Isopropanol, Aceton, Essigester, Chloroform.

Gemäß einem Beispiel ist in dem Material anfangs im Wesentlichen keine Nachweissubstanz enthalten.

Zum Beispiel handelt es sich bei dem Material um mindestens eines der folgenden: ein viskoelastisches Material, ein hochviskoses Material, eine Knetmasse, einen Haftklebstoff, eine Haftklebemasse, einen Haftklebestreifen mit einem Haftklebstoff, ein Haftklebeband mit einem Haftklebstoff, ein Haftetikett mit einem Haftklebstoff.

Entsprechend einem weiteren Beispiel umfasst das Material mindestens eines der folgenden: Acrylat, Polyvinylpyrolidon, Polyäthylenglykol, Harz sowie Abmischungen davon.

Entsprechend einem Beispiel wird die in der Beschichtung enthaltene Nachweissubstanz während der vorbestimmten Zeitspanne ganz oder teilweise aus der Beschichtung herausgelöst und tritt in das Material ein.

Gemäß einer vorteilhaften Ausführungsform der Erfindung ist die charakteristische Größe ein Maß für eine Menge oder Konzentration der Nachweissubstanz in dem Material nach Ablauf der vorbestimmten Zeitspanne.

Gemäß einem Beispiel wird nach Ablauf der vorbestimmten Zeitspanne die in dem Material enthaltene Menge oder Konzentration der Nachweissubstanz quantisiert.

Gemäß einer vorteilhaften Ausführungsform der Erfindung wird die charakteristische Größe mehrfach für verschiedene Werte der vorbestimmten Zeitspanne bestimmt.

Gemäß einer vorteilhaften Ausführungsform der Erfindung wird die charakteristische Größe als Funktion der vorbestimmten Zeitspanne bestimmt.

Erfindungsgemäß wird anhand der charakteristischen Größe eine Austrittskinetik der Nachweissubstanz in das Material bestimmt.

Erfindungsgemäß ist der Aushärtungsgrad der Beschichtung umso höher, je geringer die Menge oder Konzentration der Nachweissubstanz ist, die während der vorbestimmten Zeitspanne in das Material eintritt.

Beispielsweise handelt es sich bei der Beschichtung um eine der folgenden: eine Lackschicht, eine Druckfarbenschicht, eine Klebstoffschicht, eine Silikonisierung.

Gemäß einem Beispiel handelt es sich bei der Beschichtung um eine Schicht einer strahlungshärtenden Druckfarbe oder eines strahlungshärtenden Lackes.

Gemäß einem weiteren Beispiel ist die Beschichtung unter anderem in Form einer Druckmarke auf dem Substrat aufgebracht.

Beispielsweise handelt es sich bei der Nachweissubstanz um eine der folgenden: einen Photoinitiator, einen fluoreszenzaktiven Stoff, einen farbigen Stoff, einen Farbstoff, Farbpigmente, eine UV-aktive Substanz.

Gemäß der Erfindung wird zur Bestimmung der charakteristischen Größe mindestens eine von einer optischen Untersuchung, einer spektroskopischen Untersuchung, einer chromatographischen Untersuchung des Materials durchgeführt.

Zum Beispiel handelt es sich bei der spektroskopischen Untersuchung um eine der folgenden: UV-Spektroskopie, Spektroskopie im sichtbaren Bereich, IR-Spektroskopie, Raman-Spektroskopie, Fluoreszenzspektroskopie.

Gemäß einem Ausführungsbeispiel handelt es sich bei der Nachweissubstanz um Iod.

Zum Beispiel wird nach Ablauf der Zeitspanne eine Menge oder eine Konzentration von Iod in der Lösung bestimmt.

Beispielsweise wird das Iod, das aus der Beschichtung herausgelöst und in dem Material enthalten ist, als Iod-Stärke-Komplex nachgewiesen.

Gemäß einem Beispiel wird das Verfahren zur Bestimmung eines Anteils von migrierfähigen Substanzen in der Beschichtung eingesetzt.

Zum Beispiel ist eine Analysevorrichtung zur Bestimmung eines Aushärtungsgrades einer Beschichtung auf einem Substrat dazu ausgelegt, die Beschichtung während einer ersten Zeitspanne mit einem ersten Material in Kontakt zu bringen, in dem eine Nachweissubstanz enthalten ist. Des weiteren ist die Analysevorrichtung beispielsweise dazu ausgelegt, eine charakteristische Größe zu bestimmen, die von der während der ersten Zeitspanne in die Beschichtung eingedrungenen Menge oder Konzentration der Nachweissubstanz abhängt, und den Aushärtungsgrades der Beschichtung aus der so bestimmten charakteristischen Größe abzuleiten.

Gemäß einem Beispiel umfasst die Analysevorrichtung eine Handhabungseinheit, welche die Beschichtung während der ersten Zeitspanne in eine erste Lösung taucht.

Beispielsweise umfasst die Analysevorrichtung eine Handhabungseinheit, welche die Beschichtung während der ersten Zeitspanne in eine erste Lösung taucht, anschließend herausnimmt und analysiert.

Zum Beispiel ist eine Analysevorrichtung zur Bestimmung eines Aushärtungsgrades einer Beschichtung auf einem Substrat dazu ausgelegt, die Beschichtung während einer vorbestimmten Zeitspanne mit einem Material in Kontakt zu bringen, wobei in der Beschichtung eine vorbestimmte Menge einer Nachweissubstanz enthalten ist, und wobei das Material dazu ausgelegt ist, Nachweissubstanz aus der Beschichtung aufzunehmen. Des Weiteren ist die Analysevorrichtung beispielsweise dazu ausgelegt, eine charakteristische Größe zu bestimmen, die von der während der vorbestimmten Zeitspanne von der Beschichtung in das Material eingetretenen Menge oder Konzentration der Nachweissubstanz abhängt, und den Aushärtungsgrad der Beschichtung aus der so bestimmten charakteristischen Größe abzuleiten.

Nachfolgend wird die Erfindung anhand mehrerer in der Zeichnung dargestellter Ausführungsbeispiele weiter beschrieben.
Fig. 1 zeigt eine Ausführungsform der Erfindung zur Bestimmung der Eintrittskinetik;
Fig. 2 zeigt die Konzentration der Nachweissubstanz in der Beschichtung als Funktion der Zeit für zwei verschiedene Aushärtungsgrade;
Fig. 3 zeigt eine weitere Ausführungsform der Erfindung zur Bestimmung der Eintrittskinetik;
Fig. 4 zeigt, wie die Nachweissubstanz mittels eines Haftklebebands in die Beschichtung eingebracht werden kann;
Fig. 5 zeigt, wie die Konzentration bzw. Menge der in der Beschichtung enthaltene Nachweissubstanz mittels eines Haftklebebands bestimmt werden kann;
Fig. 6 zeigt eine Ausführungsform der Erfindung zur Bestimmung der Austrittskinetik; und
Fig. 7 zeigt die Konzentration der Nachweissubstanz in der Lösung als Funktion der Zeit für zwei verschiedene Aushärtungsgrade.

Wenn auf ein Trägermaterial eine Beschichtung aufgebracht wird, beispielsweise eine Lackschicht, eine Klebstoffschicht, eine Silikonisierung oder eine Druckfarbenschicht, insbesondere eine strahlungshärtende Druckfarbenschicht oder eine strahlungshärtende Lackschicht, dann ist es notwendig, den Aushärtungsgrad der jeweiligen Beschichtung zu bestimmen. Beispielsweise sollte vor der Weiterverarbeitung des beschichteten Substrats sichergestellt werden, dass sich der Aushärtungsgrad der Beschichtung in einem gewünschten Bereich bewegt.

Entsprechend einer ersten Ausführungsform der vorliegenden Erfindung wird der Aushärtungsgrad bestimmt, indem die Eintrittskinetik einer Nachweissubstanz in die Beschichtung bestimmt wird. Entsprechend der erfindungsgemäßen Lösung erlaubt die Eintrittskinetik einen Rückschluss auf den Aushärtungsgrad der Beschichtung. Eine gut gehärtete Beschichtung ist relativ stark vernetzt, so dass die Nachweissubstanz nur vergleichsweise langsam eindringen kann. Dagegen kann die Nachweissubstanz in eine weniger gut gehärtete Beschichtung relativ schnell eindringen.

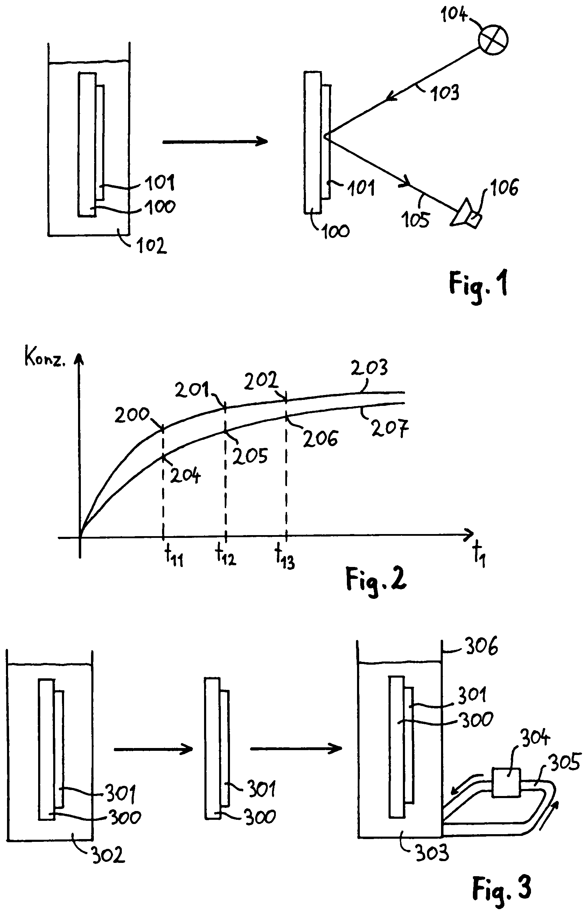

In Fig. 1 ist ein erfindungsgemäßes Verfahren zur Bestimmung der Eintrittskinetik in eine Beschichtung schematisch dargestellt. Dabei wird das Trägermaterial 100 mit der darauf befindlichen Beschichtung 101 in eine Lösung 102 mit Nachweissubstanz getaucht. In der Beschichtung 101 befindet sich anfangs keine Nachweissubstanz, während die Lösung 102 eine vorgegebene Konzentration an Nachweissubstanz enthält.

Während einer ersten Zeitspanne t1 wird das Trägermaterial 100 mit der Beschichtung 101 in die Lösung 102 getaucht, und während dieser ersten Zeitspanne t1 kann Nachweissubstanz aus der Lösung 102 in die Beschichtung 101 eindringen.

Nach Ablauf der ersten Zeitspanne t1 wird das Trägermaterial 100 mit der Beschichtung 101 aus der Lösung 102 herausgenommen und untersucht, um auf diese Weise die Menge bzw. Konzentration an Nachweissubstanz zu bestimmen, die in die Beschichtung 101 eingedrungen ist. Die Konzentration der Nachweissubstanz in der Beschichtung 101 kann z. B. mit Hilfe von optischen, spektroskopischen oder chromatographischen Untersuchungsmethoden bestimmt werden. Beispielsweise kann es sich bei der Nachweissubstanz um einen fluoreszenzaktiven Stoff, einen farbigen Stoff, einen Farbstoff, Farbpigmente, oder um eine UV-aktive Substanz handeln. In Abhängigkeit von der verwendeten Nachweissubstanz kann zur Bestimmung der Menge bzw. Konzentration der Nachweissubstanz in der Beschichtung 101 beispielsweise UV-Spektroskopie, Spektroskopie im sichtbaren Bereich, IR-Spektroskopie, Raman-Spektroskopie, oder Fluoreszenzspektroskopie eingesetzt werden.

In der rechten Hälfte von Fig. 1 ist veranschaulicht, wie die Beschichtung 101 mit spektroskopischen Verfahren untersucht wird. Die Beschichtung 101 wird mit der breitbandigen Strahlung 103 einer Strahlungsquelle 104 beaufschlagt, und die Intensität der reflektierten Strahlung 105 wird von einem Detektor 106 in Abhängigkeit von der Wellenlänge ausgewertet. Dabei absorbiert die Nachweissubstanz Strahlung bei bestimmten vorbekannten Wellenlängen, und zwar um so stärker, je höher die Konzentration der Nachweissubstanz in der Beschichtung 101 ist. Aus der vom Detektor 106 aufgezeichneten Intensität lässt sich daher zumindest näherungsweise die Menge an Nachweissubstanz in der Beschichtung 101 bestimmen, die ihrerseits wiederum einen Rückschluss auf den Aushärtungsgrad der Beschichtung 101 erlaubt.

Zur Bestimmung des Aushärtegrades kann es ausreichend sein, die Menge bzw. Konzentration der in die Beschichtung 101 während der ersten Zeitspanne t1 eingedrungenen Nachweissubstanz für eine bestimmte Zeitspanne t1 zu bestimmen. Beispielsweise kann so ermittelt werden, ob die eingedrungene Menge an Nachweissubstanz innerhalb eines vorgegebenen Toleranzfensters liegt, das dem gewünschten Aushärtungsgrad entspricht.

Alternativ dazu kann die Eintrittskinetik der Nachweissubstanz auf umfassendere Weise ermittelt werden, indem die in die Beschichtung eingedrungene Menge an Nachweissubstanz für verschiedene Zeitspannen t1 bestimmt wird. Dazu können beispielswiese mehrere gleiche Proben gleichzeitig in die Lösung 102 eingebracht und jeweils nach verschiedenen Zeitspannen t11, t12, t13, etc. wieder entnommen und untersucht werden.

Als Ergebnis einer derartigen Untersuchung ergibt sich die Menge bzw. Konzentration der Nachweissubstanz in der Beschichtung in Abhängigkeit von der Eindringzeit t1.

Ein auf diese Weise erhaltenes Ergebnis ist in Fig. 2 graphisch dargestellt. Bei einer relativ schlecht ausgehärteten Beschichtung werden zu den Zeitspannen t11, t12, t13 die Messwerte 200, 201, 202 ermittelt, die zu jeder Zeitspanne t11, t12, t13 die in die Beschichtung eingedrungene Menge an Nachweissubstanz angeben. Die Messwerte 200, 201, 202 legen eine Kurve 203 fest, die das Eindringverhalten der Nachweissubstanz in die Beschichtung als Funktion der Einwirkzeit t1 spezifiziert. Bei einer relativ gut ausgehärteten Beschichtung werden zu den Zeitspannen t11, t12, t13 die Messwerte 204, 205, 206 ermittelt, die zu jeder Zeitspanne t11, t12, t13 die eingedrungene Menge an Nachweissubstanz angeben. Die durch die Messwerte 204, 205, 206 festgelegte Kurve 207 veranschaulicht das Eindringverhalten der Nachweissubstanz in die relativ gut ausgehärtete Beschichtung. Aus dem Vergleich der Kurven 203 und 207 ergibt sich, dass die Nachweissubstanz in eine relativ schlecht ausgehärtete Beschichtung deutlich schneller eindringt als in eine vergleichsweise gut ausgehärtete Beschichtung.

Zur Verwendung als Nachweissubstanz hat sich insbesondere die Verwendung von Iod als vorteilhaft erwiesen. Wegen der charakteristischen Gelbfärbung von Iod lässt sich das in die Beschichtung eingedrungene Iod spektroskopisch leichtnachweisen. Darüber hinaus kann das in die Beschichtung eingedrungene Iod auch zu Iod-Stärke-Komplexen umgesetzt werden, die sich wegen ihrer charakteristischen Blaufärbung einfach nachweisen lassen.

Ein alternatives Verfahren zur Bestimmung der Eintrittskinetik der Nachweissubstanz in die Beschichtung ist in Fig. 3 gezeigt. Bei dem in Fig. 3 gezeigten Verfahren wird ein Trägermaterial 300 mit einer darauf aufgebrachten Beschichtung 301 während einer ersten Zeitspanne t1 in eine erste Lösung 302 eingebracht. Die erste Lösung 302 enthält eine vorgegebene Konzentration einer Nachweissubstanz, während die Beschichtung 301 anfangs keine Nachweissubstanz enthält. Während der ersten Zeitspanne t1 dringt Nachweissubstanz aus der ersten Lösung 302 in die Beschichtung 301 ein.

Nach Ablauf der ersten Zeitspanne t1 wird das Trägermaterial 300 mit der Beschichtung 301 aus der ersten Lösung 300 herausgenommen und daraufhin zur Auswertung in eine zweite Lösung 303 eingebracht. Die zweite Lösung 303 enthält anfangs keine Nachweissubstanz. Das Trägermaterial 300 mit der Beschichtung 301 verbleibt während einer zweiten Zeitspanne t2 in der zweiten Lösung, und während dieser zweiten Zeitspanne t2 tritt die in der Beschichtung 301 enthaltene Nachweissubstanz zumindest zum Teil in die zweite Lösung 303 ein. Nach Ablauf der zweiten Zeitspanne t2 kann die Konzentration von Nachweissubstanz in der zweiten Lösung 303 mit Hilfe eines Detektors 304 ermittelt werden, der beispielsweise über ein Kreislaufsystem 305 mit dem Behälter 306, der die zweite Lösung 303 enthält, gekoppelt ist.

Gemäß einer vorteilhaften Ausführungsform ist die zweite Zeitspanne t2 so lang gewählt, dass die in der Beschichtung 301 enthaltene Nachweissubstanz vollständig oder annähernd vollständig in die zweite Lösung 303 eintritt. Nach dem Ablauf der zweiten Zeitspanne t2 kann mit Hilfe des Detektors 304 die Konzentration der Nachweissubstanz in der zweiten Lösung 303 ermittelt werden. Auf diese Weise kann ermittelt werden, welche Menge an Nachweissubstanz während der ersten Zeitspanne t1 in die Beschichtung eingedrungen ist. Auf diese Weise kann die Eintrittskinetik der Nachweissubstanz ermittelt werden.

Falls das Eintritts- und Austrittsgeschwindigkeit für die Nachweissubstanz relativ langsam ist, muss die zweite Zeitspanne t2 nicht notwendigerweise so groß gewählt werden, dass nach Ablauf von t2 annähernd die gesamte in der Beschichtung 301 enthaltene Nachweissubstanz in die zweite Lösung 303 übergetreten ist. Stattdessen kann die zweite Zeitspanne t2 auch kürzer gewählt werden. In diesem Fall schließt sich an die durch die erste Zeitspanne t1 festgelegte (unvollständige) Eintrittskinetik eine durch die Zeitspanne t2 festgelegte, ebenfalls unvollständige Austrittskinetik an. Nach Ablauf der zweiten Zeitspanne wird die Konzentration der Nachweissubstanz in der zweiten Lösung 303 ermittelt. Sowohl für die Eintrittskinetik als auch für die Austrittskinetik ergibt sich mit zunehmender Aushärtung der Beschichtung 301 eine Verlangsamung der Eintritts- bzw. Austrittsgeschwindigkeit. Sowohl das verlangsamte Eintrittsverhalten als auch das verlangsamte Austrittsverhalten führen jeweils zu einer Verringerung der Menge an Nachweissubstanz, die sich nach Ablauf der zweiten Zeitspanne t2 in der zweiten Lösung 303 befindet. Insofern wirken beide Effekte in dieselbe Richtung, und deshalb kann der Aushärtungsgrad der Beschichtung auch bei unvollständiger Austrittskinetik mit guter Sensitivität bestimmt werden.

Anstatt die Nachweissubstanz in die Beschichtung einzubringen, indem das Trägermaterial mit der Beschichtung in eine Lösung von Nachweissubstanz getaucht wird, kann die Nachweissubstanz auch durch Aufkleben eines Haftetiketts oder eines Haftklebebands in die Beschichtung eingebracht werden. Hierzu weist der Haftklebstoff des Haftetiketts oder -klebebands eine gewisse Konzentration einer Nachweissubstanz auf, die nach dem Aufkleben des Haftetiketts in die Beschichtung des Trägermaterials eindringt.

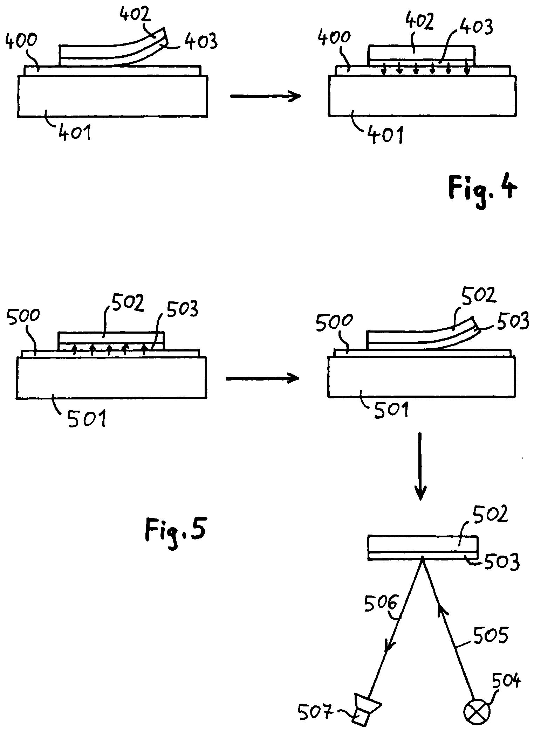

Eine derartige Ausführungsform ist in Fig. 4 gezeigt. Auf eine Beschichtung 400, die sich auf einem Trägermaterial 401 befindet, wird ein Haftklebeband 402 aufgeklebt, das eine Haftklebstoffschicht 403 aufweist. Die Haftklebstoffschicht 403 kann beispielsweise aus Acrylat, Polyvinylpyrolidon, Polyäthylenglykol, Harz sowie Abmischungen davon bestehen. Die Haftklebstoffschicht 403 umfasst eine gewisse Konzentration einer Nachweissubstanz. Beispielsweise kann die Haftklebstoffschicht 403 eine gewisse Konzentration an Iod aufweisen. Nach dem Aufkleben des Haftklebebands 402 auf die Beschichtung 400 dringt die in der Haftklebstoffschicht 403 enthaltene Nachweissubstanz in die Beschichtung 400 ein.

Zum Aufbringen der Nachweissubstanz wird das Haftklebeband 402 für eine erste Zeitspanne t1 auf die Beschichtung 400 aufgeklebt und anschließend wieder abgezogen. Auf diese Weise kann erreicht werden, dass die Nachweissubstanz während einer ersten Zeitspanne t1 in die Beschichtung 400 eindringt. Wenn die Nachweissubstanz entsprechend der in Fig. 4 gezeigten Ausführungsform mittels eines Haftklebebands aufgebracht wird, dann hat das unter anderem den Vorteil, dass das Haftklebeband gezielt auf speziell zu diesem Zweck auf dem Trägermaterial 401 vorgesehene Druckmarken aufgebracht werden kann.

Auch zur Bestimmung der Menge oder Konzentration der in die Beschichtung eingedrungenen Nachweissubstanz kann anstelle der in Fig. 3 gezeigten zweiten Lösung 303 ein Haftklebeband verwendet werden. Eine derartige Ausführungsform ist in Fig. 5 gezeigt. In der Beschichtung 500 auf dem Trägermaterial 501 befindet sich eine zu bestimmende Menge oder Konzentration an Nachweissubstanz. Zur Bestimmung der Menge oder Konzentration der Nachweissubstanz wird ein Haftklebeband 502 mit einer Haftklebstoffschicht 503 auf die Beschichtung 500 aufgeklebt und dort für eine bestimmte zweite Zeitspanne t2 belassen. Die Haftklebstoffschicht 503 enthält anfangs keine Nachweissubstanz. Während der zweiten Zeitspanne t2 tritt Nachweissubstanz aus der Beschichtung 500 in die Haftklebstoffschicht 503 ein. Nach Ablauf der zweiten Zeitspanne t2 wird das Haftklebeband 502 von der Beschichtung 500 abgezogen. Die Haftklebstoffschicht 503 enthält nun eine gewisse Menge an Nachweissubstanz, wobei die Menge an Nachweissubstanz vom Aushärtungsgrad der Beschichtung 500 abhängt. Bei relativ gutem Aushärtungsgrad ist die Menge an Nachweissubstanz in der Haftklebstoffschicht 503 eher gering, während bei unvollständiger Aushärtung der Beschichtung 500 eine größere Menge an Nachweissubstanz in die Haftklebstoffschicht 503 eindringt. Die Bestimmung der in die Haftklebstoffschicht 503 eingedrungenen Menge an Nachweissubstanz erlaubt daher einen Rückschluss auf den Aushärtungsgrad der Beschichtung 500. Die Verwendung eines Haftklebebands bietet unter anderem den Vorteil, dass das Haftklebeband gezielt auf eine speziell hierfür auf dem Trägermaterial vorgesehene Druckmarke aufgeklebt werden kann.

Die Bestimmung der in die Haftklebstoffschicht 503 eingedrungenen Menge an Nachweissubstanz kann beispielsweise, wie in Fig. 5 gezeigt, durch spektroskopische Methoden erfolgen. Hierzu wird die Haftklebstoffschicht 503 durch eine von einer Strahlungsquelle 504 erzeugte breitbandige Strahlung 505 bestrahlt, und die reflektierte Strahlung 506 wird von einem Detektor 507 analysiert. Beispielsweise kann im Detektor 507 aus der Stärke von Absorptionsbanden die Menge bzw. Konzentration von Nachweissubstanz in der Haftklebstoffschicht 503 bestimmt werden.

Entsprechend einer weiteren Ausführungsform der vorliegenden Erfindung wird der Aushärtungsgrad bestimmt, indem die Austrittskinetik einer Nachweissubstanz bestimmt wird, wobei die Nachweissubstanz bereits zu Beginn des Verfahrens in der Beschichtung enthalten ist. Entsprechend der erfindungsgemäßen Lösung erlaubt die Austrittskinetik einen Rückschluss auf den Aushärtungsgrad der Beschichtung. Eine gut gehärtete Beschichtung ist relativ stark vernetzt, so dass die Nachweissubstanz nur vergleichsweise langsam aus der Beschichtung austreten kann. Dagegen kann aus einer weniger gut gehärteten Beschichtung die Nachweissubstanz relativ schnell austreten.

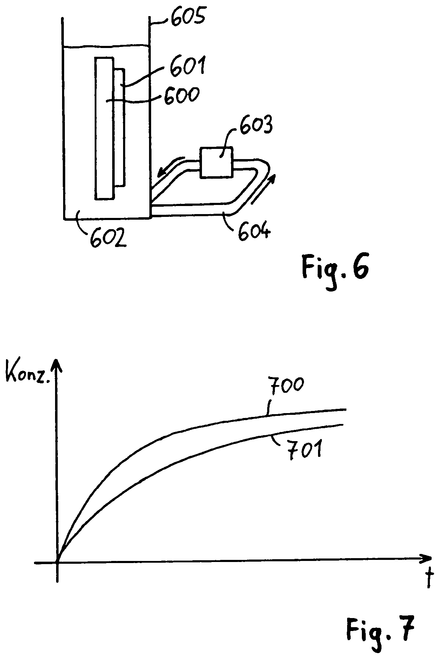

In Fig. 6 ist ein erfindungsgemäßes Verfahren zur Bestimmung der Austrittskinetik aus einer Beschichtung schematisch dargestellt. Das Trägermaterial 600 umfasst eine Beschichtung 601, die bereits zu Beginn des Verfahrens eine vorgegebene Menge oder Konzentration von Nachweissubstanz enthält. Bei der Nachweissubstanz kann es sich beispielsweise um einen fluoreszenzaktiven Stoff, einen farbigen Stoff, einen Farbstoff, Farbpigmente, oder eine UV-aktive Substanz handeln. Bei der Nachweissubstanz kann es sich aber auch um einen Photoinitiator handeln, der zur Polymerisation der Beschichtung 601 eingesetzt wurde und sich daher noch in der Beschichtung 601 befindet.

Zur Bestimmung der Austrittskinetik der Nachweissubstanz aus der Beschichtung 601 wird das Trägermaterial 600 mit der Beschichtung 601 in eine Lösung 602 gegeben, welche anfangs keinerlei Nachweissubstanz enthält. Ab dem Einbringen der Beschichtung 601 in die Lösung 602 tritt Nachweissubstanz aus der Beschichtung 601 in die Lösung 602 ein, und die Menge bzw. Konzentration der Nachweissubstanz in der Lösung 602 steigt an. Die aktuelle Konzentration der Nachweissubstanz in der Lösung 602 kann mittels eines Detektors 603 bestimmt werden, der beispielsweise über ein Kreislaufsystem 604 mit dem Behälter 605, der die Lösung 602 enthält, gekoppelt ist. Auf diese Weise kann die Menge oder Konzentration der Nachweissubstanz in der Lösung 602 entweder kontinuierlich oder aber nach Ablauf einer vordefinierten Zeitspanne ermittelt werden, um auf diese Weise die Austrittsgeschwindigkeit der Nachweissubstanz aus der Beschichtung 601 zu bestimmen.

In Fig. 7 ist die Menge bzw. Konzentration von Nachweissubstanz in der Lösung 602 als Funktion der Zeit t für zwei verschiedene Aushärtungsgrade der Beschichtung 601 dargestellt. Dabei zeigt die Kurve 700 einen vergleichsweise schnellen Anstieg der Menge bzw. Konzentration von Nachweissubstanz in der Lösung 602. Die Kurve 700 entspricht daher einer eher unvollständigen Aushärtung der Beschichtung 601. Dagegen zeigt die Kurve 701 einen vergleichsweise langsamen Anstieg der Menge bzw. Konzentration von Nachweissubstanz in der Lösung 602. Die Kurve 701 entspricht daher einem guten Aushärtungsgrad der Beschichtung 601.

## Patentansprüche

1. Verfahren zur Bestimmung eines Aushärtungsgrades einer Beschichtung (101, 301) auf einem Substrat (100, 300), wobei das Verfahren umfasst:
a) in Kontakt bringen der Beschichtung (101, 301) während einer ersten Zeitspanne mit einem ersten Material (102, 302), das eine vorgegebene Konzentration an Nachweissubstanz enthält,
b) quantitatives Bestimmen einer charakteristischen Größe, die von einer während der ersten Zeitspanne in die Beschichtung (101, 301) eingedrungenen Menge oder Konzentration der Nachweissubstanz abhängt, mittels mindestens einer von einer optischen Untersuchung, einer spektroskopischen Untersuchung, einer chromatographischen Untersuchung der Beschichtung (101, 301),
c) Bestimmen einer Eintrittskinetik der Nachweissubstanz in die Beschichtung (101, 301) anhand der charakteristischen Größe, wobei eine gut gehärtete Beschichtung relativ stark vernetzt ist, so dass die Nachweissubstanz nur vergleichsweise langsam eindringen kann, wohingegen die Nachweissubstanz in eine weniger gut gehärtete Beschichtung relativ schnell eindringen kann, wobei die Eintrittskinetik einen Rückschluss auf den Aushärtungsgrad der Beschichtung erlaubt,
d) Ableiten des Aushärtungsgrades der Beschichtung (101, 301) aus der Eintrittskinetik der Nachweissubstanz in die Beschichtung (101, 301), wobei der Aushärtungsgrad der Beschichtung umso höher ist, je geringer die Menge oder Konzentration der Nachweissubstanz ist, die während der ersten Zeitspanne in die Beschichtung (101, 301) eindringt.

2. Verfahren nach Anspruch 1, **dadurch gekennzeichnet, dass** die charakteristische Größe ein Maß ist für eine Menge oder Konzentration der Nachweissubstanz in der Beschichtung nach Ablauf der ersten Zeitspanne.

3. Verfahren nach Anspruch 1 oder Anspruch 2, **dadurch gekennzeichnet, dass** die charakteristische Größe mehrfach für verschiedene Werte der ersten Zeitspanne bestimmt wird.

4. Verfahren nach einem der Ansprüche 1 bis 3, **dadurch gekennzeichnet, dass** die charakteristische Größe als Funktion der ersten Zeitspanne bestimmt wird.

5. Verfahren nach einem der Ansprüche 1 bis 4, **gekennzeichnet durch**:
in Kontakt bringen, nach Ablauf der ersten Zeitspanne, der Beschichtung mit einem zweiten Material während einer zweiten Zeitspanne, wobei das zweite Material dazu ausgelegt ist, Nachweissubstanz aus der Beschichtung aufzunehmen, Bestimmen der charakteristischen Größe als Maß für eine Menge oder Konzentration der Nachweissubstanz in dem zweiten Material.

6. Verfahren zur Bestimmung eines Aushärtungsgrades einer Beschichtung (601) auf einem Substrat (600), wobei das Verfahren umfasst:
a) in Kontakt bringen der Beschichtung (601) während einer vorbestimmten Zeitspanne mit einem Material (602), wobei in der Beschichtung (601) eine vorbestimmte Menge einer Nachweissubstanz enthalten ist, und wobei das Material (602) dazu ausgelegt ist, Nachweissubstanz aus der Beschichtung (601) aufzunehmen,
b) quantitatives Bestimmen einer charakteristischen Größe, die von einer während der vorbestimmten Zeitspanne von der Beschichtung (601) in das Material (602) eingetretenen Menge oder Konzentration der Nachweissubstanz abhängt, mittels mindestens einer von einer optischen Untersuchung, einer spektroskopischen Untersuchung, einer chromatographischen Untersuchung des Materials (602),
c) Bestimmen einer Austrittskinetik der Nachweissubstanz in das Material (602) anhand der charakteristischen Größe, wobei eine gut gehärtete Beschichtung relativ stark vernetzt ist, so dass die Nachweissubstanz nur vergleichsweise langsam aus der Beschichtung austreten kann, wohingegen aus einer weniger gut gehärteten Beschichtung die Nachweissubstanz relativ schnell austreten kann, wobei die Austrittskinetik einen Rückschluss auf den Aushärtungsgrad der Beschichtung (601) erlaubt,
d) Ableiten des Aushärtungsgrades der Beschichtung (601) aus der Austrittskinetik der Nachweissubstanz, wobei der Aushärtungsgrad der Beschichtung (601) umso höher ist, je geringer die Menge oder Konzentration der Nachweissubstanz ist, die während der vorbestimmten Zeitspanne in das Material (602) eintritt.

7. Verfahren nach Anspruch 6, **dadurch gekennzeichnet, dass** die charakteristische Größe ein Maß ist für eine Menge oder Konzentration der Nachweissubstanz in dem Material nach Ablauf der vorbestimmten Zeitspanne.

8. Verfahren nach Anspruch 6 oder Anspruch 7, **dadurch gekennzeichnet, dass** die charakteristische Größe mehrfach für verschiedene Werte der vorbestimmten Zeitspanne bestimmt wird.

9. Verfahren nach einem der Ansprüche 6 bis 8, **dadurch gekennzeichnet, dass** die charakteristische Größe als Funktion der vorbestimmten Zeitspanne bestimmt wird.

## Claims

1. A method for determining the degree of curing of a coating (101, 301) on a substrate (100, 300), wherein the method comprises:
a) bringing the coating (101, 301) into contact, over a first period of time, with a first material (102, 302) containing a predetermined concentration of detection substance,
b) quantitatively determining a characteristic quantity, which depends on the amount or concentration of the detection substance which has penetrated into the coating (101, 301) over the first period of time, by means of at least one of a visual investigation, a spectroscopic investigation or a chromatographic investigation of the coating (101, 301),
c) determining entry kinetics for entry of the detection substance into the coating (101, 301) on the basis of the characteristic quantity, wherein a well cured coating is relatively highly crosslinked, such that the detection substance can penetrate only comparatively slowly, whereas the detection substance can penetrate relatively quickly into a less well cured coating, wherein the entry kinetics allows a conclusion to be drawn as the degree of curing of the coating,
d) deducing the degree of curing of the coating (101, 301) from the entry kinetics for entry of the detection substance into the coating (101, 301), wherein the degree of curing of the coating is greater, the lower the amount or concentration of the detection substance which penetrates into the coating (101, 301) over the first period of time.

2. A method according to claim 1, **characterised in that** the characteristic quantity is a measure of an amount or concentration of the detection substance in the coating once the first period of time has elapsed.

3. A method according to claim 1 or claim 2, **characterised in that** the characteristic quantity is determined repeatedly for different values of the first period of time.

4. A method according to one of claims 1 to 3, **characterised in that** the characteristic quantity is determined as a function of the first period of time.

5. A method according to one of claims 1 to 4, **characterised by**:
bringing the coating into contact with a second material over a second period of time, once the first period of time has elapsed, wherein the second material is designed to absorb detection substance from the coating and
determining the characteristic quantity as a measure of an amount or concentration of the detection substance in the second material.

6. A method for determining the degree of curing of a coating (601) on a substrate (600), wherein the method comprises:
a) bringing the coating (601) into contact, over a predetermined period of time, with a material (602), wherein the coating (601) contains a predetermined amount of a detection substance and wherein the material (602) is designed to absorb detection substance from the coating (601),
b) quantitatively determining a characteristic quantity, which depends on the amount or concentration of the detection substance which entered into the material (602) from the coating (601) over the predetermined period of time, by means of at least one of a visual investigation, a spectroscopic investigation or a chromatographic investigation of the material (602),
c) determining exit kinetics for exit of the detection substance into the material (602) on the basis of the characteristic quantity, wherein a well cured coating is relatively highly crosslinked, such that the detection substance can exit from the coating only comparatively slowly, whereas the detection substance can exit relatively quickly from a less well cured coating, wherein the exit kinetics allows a conclusion to be drawn as the degree of curing of the coating (601),
d) deducing the degree of curing of the coating (601) from the exit kinetics of the detection substance, wherein the degree of curing of the coating (601) is greater, the lower the amount or concentration of the detection substance which enters into the material (602) over the predetermined period of time.

7. A method according to claim 6, **characterised in that** the characteristic quantity is a measure of an amount or concentration of the detection substance in the material once the predetermined period of time has elapsed.

8. A method according to claim 6 or claim 7, **characterised in that** the characteristic quantity is determined repeatedly for different values of the predetermined period of time.

9. A method according to one of claims 6 to 8, **characterised in that** the characteristic quantity is determined as a function of the predetermined period of time.

## Revendications

1. Procédé pour déterminer un degré de durcissement d'un revêtement (101, 301) sur un substrat (100, 300), le procédé comprenant les étapes suivantes :
a) mise en contact du revêtement (101, 301) pendant une première période de temps avec un premier matériau (102, 302) contenant une concentration prédéterminée en une substance de détection,
b) détermination quantitative d'une variable caractéristique dépendant de la quantité ou de la concentration de la substance de détection ayant pénétré dans le revêtement (101, 301) pendant une première période de temps, au moyen d'au moins un des examens optique, spectroscopique, chromatographique du revêtement (101, 301),
c) détermination d'une cinétique d'entrée de la substance de détection dans le revêtement (101, 301) au moyen de la variable caractéristique, un revêtement bien durci étant relativement fortement réticulé, de sorte que la substance de détection ne peut pénétrer que relativement lentement, la substance de détection pouvant par contre pénétrer relativement rapidement dans un revêtement moins bien durci, la cinétique d'entrée permettant de déduire le degré de durcissement du revêtement,
d) déduction du degré de durcissement du revêtement (101, 301) à partir de la cinématique d'entrée de la substance de détection dans le revêtement (101, 301), le degré de durcissement du revêtement étant d'autant plus élevé que la quantité ou la concentration de la substance de détection pénétrant dans le revêtement (101, 301) pendant la première période de temps, est faible.

2. Procédé selon la revendication 1, **caractérisé en ce que** la variable caractéristique est une mesure de la quantité ou de la concentration de la substance de détection dans le revêtement au terme de la première période de temps.

3. Procédé selon la revendication 1 ou la revendication 2, **caractérisé en ce que** la variable caractéristique est déterminée plusieurs fois pour plusieurs valeurs de la première période de temps.

4. Procédé selon une des revendications 1 à 3, **caractérisé en ce que** la variable caractéristique est déterminée comme fonction de la première période de temps.

5. Procédé selon une des revendications 1 à 4, **caractérisé par** :
mise en contact, au terme de la première période de temps du revêtement avec un deuxième matériau pendant une deuxième période de temps, le deuxième matériau étant de nature à absorber la substance de détection à partir du revêtement, détermination de la variable caractéristique comme mesure d'une quantité ou concentration de la substance de détection dans le deuxième matériau.

6. Procédé pour la détermination d'un degré de durcissement d'un revêtement (601) sur un substrat (600), le procédé comprenant les étapes suivantes :
a) mise en contact du revêtement (601) pendant une période de temps prédéterminée avec un matériau (602,) le revêtement (601) contenant une quantité prédéterminée d'une substance de détection et le matériau (602) étant de nature à absorber la substance de détection à partir du revêtement (601),
b) détermination quantitative d'une variable caractéristique dépendant de la quantité ou de la concentration de la substance de détection ayant passé du revêtement (601) dans le matériau (602) pendant la période de temps prédéterminées, au moyen d'au moins un des examens optique, spectroscopique, chromatographique du matériau (602),
c) détermination de la cinétique de sortie de la substance de détection dans le matériau (602) au moyen de la variable caractéristique, un revêtement relativement bien durci étant relativement fortement réticulé, de sorte que la substance de détection ne peut sortir du revêtement que relativement lentement, la substance de détection pouvant par contre sortir relativement rapidement d'un revêtement moins bien durci, la cinétique de sortie permettant de déduire le degré de durcissement du revêtement (601),
d) déduction du degré de durcissement du revêtement (601) de la cinétique de sortie de la substance de détection, le degré de durcissement du revêtement (601) étant d'autant plus élevé que la quantité ou la concentration de la substance de détection pénétrant dans le matériau (602) pendant la période de temps prédéterminée, est faible.

7. Procédé selon la revendication 6, **caractérisé en ce que** la variable caractéristique est une mesure pour une quantité ou une concentration de la substance de détection dans le matériau au terme de la période de temps prédéterminée.

8. Procédé selon la revendication 6 ou la revendication 7, **caractérisé en ce que** la variable caractéristique est déterminée plusieurs fois pour différentes valeurs de la période de temps prédéterminée.

9. Procédé selon une des revendications 6 à 8, **caractérisé en ce que** la variable caractéristique est déterminée comme fonction de la période de temps prédéterminée.
